# EUROPÄISCHE PATENTSCHRIFT

(11) **EP 0 664 131 B1**
(45) Veröffentlichungstag und Bekanntmachung des Hinweises auf die Patenterteilung: **06.06.2001**
(21) Anmeldenummer: 95100411.8
(22) Anmeldetag: 13.01.1995
(51) Int. Cl.: A61K 35/78, A61K 9/14

(54) **Optimal freisetzende Kava-Extrakte**
Kava extracts with optimal release
Extraits de kava à libération optimale

(30) Priorität: 21.01.1994 DE 4401646
(43) Veröffentlichungstag der Anmeldung: 26.07.1995
(73) Patentinhaber: Krewel Meuselbach GmbH, 53783 Eitorf (DE)
(72) Erfinder: Schierstedt, Detlef, Dr. Dipl.-Chem., D-53757 St. Augustin-Meindorf (DE)
(74) Vertreter: Jönsson, Hans-Peter, Dr.Dipl.-Chem.

(56) Entgegenhaltungen:
- EP-A- 0 523 591
- EP-A- 0 552 708
- WO-A-92/04036
- DE-A- 3 114 214
- FR-A- 2 327 764
- JOURNAL OF PHARMACEUTICAL SCIENCES, Bd. 60, no.9, September 1971 USA, Seiten 1281-1302, WIN LOUNGCHIOU ET AL 'Pharmaceutical Applications of Solid Dispersion Systems'

## Beschreibung

Gegenstand der Erfindung sind peroral applizierbare Kava-Extrakte und ein Verfahren zu ihrer Herstellung.

Präparate, die Kava-Extrakte enthalten, werden in Polynesien von Eingeborenen seit jeher zu rituellen und therapeutischen Zwecken verwendet. So sind aus der EP-0 523 591 A2 verschiedene medizinische Indikationen, beispielsweise als Phytotranquilizer zur Entspannung bei Nervosität und Überreizung als Mittel zur Schlafinduktion bekannt.

Kava-Extrakte werden in der Medizin zur Behandlung von Angst-, Spannungs- und Unruhezuständen eingesetzt. Aufgrund der starken lokal anästhesierenden Wirkung werden üblicherweise Kava-Extrakte zu Pulverzubereitungen verarbeitet und in festen peroralen Arzneiformen, beispielsweise Tabletten und Kapseln eingesetzt. Präparate, die Kava-Extrakt enthalten, unterscheiden sich von vielen anderen Phyto-Pharmaka dadurch, daß die spezifischen Wirkstoffe bekannt sind und somit die Möglichkeit besteht, auf einen bestimmten Wirkstoffgehalt zu standardisieren. Standardisiert wird üblicherweise auf die 6 Hauptkavapyrone (Desmethoxyyangonin, Dihydrokawain, Yangonin, Kawain, Dihydromethysticin und Methysticin). Daneben sind in der Kava-Droge die selben Flavokawine A und B enthalten, denen hautspezifische Nebenwirkungen zugeschrieben werden (Schulgin, Bulletin of Narcotics, Vol. XXV, Nr. 2, Seite 59-74 (1973)).

Schließlich enthält die Droge noch eine Reihe weiterer, noch nicht vollständig geklärter Inhaltsstoffe, die pharmakologisch inert und als "Matrixstoffe" bezeichnet werden.

Aus DE-31 14 214 A1 sind galenische Zubereitungen von DL-Kawain für eine verbesserte enterale Resorption als feste Lösung oder Dispersion von DL-Kawain in einem pharmazeutisch unbedenklichen festen Träger, wie einem Polyäthylenglykol, Cholesterin, Cholsäure und/oder Dehydrocholsäure bekannt. Der alleinige Wirkstoff DL-Kawain wird zusammen mit den Trägern bei hoher Temperatur geschmolzen und die Schmelze schockartig abgekühlt. Die abgekühlte Schmelze wird anschließend maschinell zu einem Pulver zerkleinert.

Die WO 92/04036 betrifft einen Trockenextrakt aus Kawa-Kawa-Droge mit einem Gesamtgehalt an Kawalactonen von mindestens 50 Gew.-% und einem Flavokawingehalt von höchstens 0,3 Gew.-%. Der Extrakt zeichnet sich durch gute Wasserlöslichkeit und hohe Bioverfügbarkeit nach oraler Applikation aus. Um den Extrakt an Kawalactonen an- und an Flavokawinen abzureichern, wird ein Verfahren vorgeschlagen, bei dem der Rohextrakt in Lösung gebracht und der Gehalt an Flavokawinen durch Kältefällung oder durch Lösungsmittelverteilung verringert wird, wonach die Lösung zur Trockne eingeengt wird. Unter Verwendung des Trockenextrakts werden Arzneimittel hergestellt.

Allen Kavapyronen und insbesondere den oben genannten 6 Hauptkavapyronen ist gemein, daß sie relativ lipophil und somit schwer wasserlöslich sind. Zwischen den einzelnen Pyronen bestehen darüberhinaus Unterschiede in ihrer Löslichkeit im Magen-Darm-Milieu. Übliche Kava-Extrakt-Pulverzubereitungen setzen daher Kavapyrone im Magen-Darmtrakt nicht vollständig frei und werden somit zu einem großen Teil unverändert wieder ausgeschieden. Unterschiedliche Mageninhalte bei der Einnahme üblicher Kava-Pulver-Zubereitungen beeinflussen darüberhinaus in einem bestimmten Rahmen das Ausmaß der Restfreisetzung. Weiterhin werden die Kavapyrone nicht gleichmäßig, sondern in unterschiedlichen Anteilen freigesetzt, so daß bei der Einnahme derartiger Präparate sowohl ein Einfluß auf die Wirkungsquantität als auch auf die Wirkungsqualität zu erwarten ist.

Die Aufgabe der vorliegenden Erfindung besteht daher darin, peroral applizierbare Kava-Extrakte zur Verfügung zu stellen, die eine hohe Freisetzungsrate von Kavapyronen im Magen-Darmtrakt aufweisen.

In einem ersten Aspekt der vorliegenden Erfindung wird die vorgenannte Aufgabe gelöst durch peroral applizierbare Kava-Extrakte, dadurch gekennzeichnet, daß sie Kavapyrone an bei Raumtemperatur feste Träger, ausgewählt aus Polyvinylpyrrolidon, Cellulosederivaten und/oder Stärkederivaten, in mikrodisperser Form und/oder in Form einer festen Lösung gebunden, gegebenenfalls neben weiteren Hilfs- und/oder Zusatzstoffen enthalten, wobei das Gewichtsverhältnis von Kavapyronen zu Träger 1:1 bis 1:10 beträgt.

Überraschenderweise wurde gefunden, daß durch Lösen und/oder Suspendieren der bei Raumtemperatur festen Träger in einem Kavafluidextrakt nach dem Einengen ein Kavaträger-Komplex entsteht, bei dem die Kavapyrone in mikrodisperser Form und/oder in Lösung gebunden sind.

Bei Messungen der Freisetzung der Kavapyrone unter Einsatz eines Mediums, das dem Magen-Darm-Milieu entspricht, wurde eine gegenüber dem Stand der Technik signifikant verbesserte Freisetzung der Kavapyrone in dem gesamten Wirkstoffspektrum der eingangs genannten 6 Hauptkavapyrone erhalten. Die Wirkstoffreisetzung aus festen peroralen Arzneiformen kann beispielsweise nach dem Deutschen Arzneibuch DAB 10 V5.4 bestimmt werden. Darüber hinaus wurde erfindungsgemäß gefunden, daß insbesondere beim Einsatz von quervernetzten Cellulosederivaten die Flavokawine signifikant im Magensaftmilieu adsorptiv gebunden werden, ohne die Freisetzung der Kavapyrone merklich zu behindern. Diese Adsorption der Flavokawine führt zu einer verminderten Resorption der Flavokawine, was bei Langzeitbehandlung, die durch diese bedingten Nebenwirkungen reduziert.

In einer bevorzugten Ausführungsform der vorliegenden Erfindung werden die quervernetzten Cellulose- und/oder Stärkederivate ausgewählt aus Alkalimetallsalzen von Carboxymethylstärke oder von Carboxymethylcellulose, wobei das Natriumsalz der Carboxymethylcellulose besonders bevorzugt wird. Diese festen Träger sind geeignet, die Flavokawine im Magensaftmilieu adsorptiv zu binden und so die nachteiligen Wirkungen zu unterdrücken.

In einer weiteren bevorzugten Ausführungsform der vorliegenden Erfindung ist der bei Raumtemperatur feste Träger Polyvinylpyrrolidon, mit einer Molekularmasse von 17.000 bis 30.000, insbesondere von 25.000. Dieses, im Handel erhältliche weiße bis gelblich-weiße, feine bis körnige Pulver, das als Verdickungsmittel, Suspensionsstabilisator, Lösungsvermittler und Bindemittel für orale Präparate bekannt ist, ist geeignet, feste Lösungen und/oder Mikrodispersionen mit den Kavapyronen zu bilden.

Bevorzugterweise wird das Gewichtsverhältnis von Kavapyronen zu den festen Trägern im Bereich von 1:2 bis 1:6 eingestellt. Wird das Verhältnis von Kavapyronen zu festen Trägern zu groß gewählt, so ist die Bindung der Kavapyrone an den festen Träger nicht ausreichend und damit keine optimale Freisetzung gegeben, während bei einem zu kleinen Verhältnis von Kavapyronen zu festen Trägern eine unnötige Volumenvergrößerung der Arzneimittelzubereitung eintritt.

Zur Herstellung von Tabletten, Filmtabletten, Dragees oder Kapseln werden die peroral applizierbaren Kava-Extrakte gegebenenfalls mit weiteren Hilfs- und/oder Zusatzstoffen versetzt. So können bei der Herstellung von Tabletten die Hilfs- und/oder Zusatzstoffe vorzugsweise ausgewählt sein aus Bindemitteln, Gleitmitteln, Füllstoffen, Emulgatoren, Netzmitteln und/oder Sprengmitteln.

Ein weiterer Aspekt der vorliegenden Erfindung umfaßt das Verfahren zur Herstellung von peroral applizierbaren Kava-Extrakten.

Zum Einsatz kommen vorzugsweise Fluidextrakte. Zu diesen wird ein, bei Raumtemperatur fester Träger, gegebenenfalls bei erhöhter Temperatur, zugegeben. Bei erhöhter Temperatur und/oder unter Vakuum wird der Fluidextrakt anschließend bis zur Trockne eingeengt. Fluidextrakte im Sinne der Erfindung umfassen auch Spissum- oder Trockenextrakte, die nach an sich bekannten Verfahren hergestellt werden und durch geeignete Lösungsmittel wieder aufgelöst werden.

Das Einengen zur Trockne kann erfindungsgemäß auch durch Sprühtrocknung nach bekannten Verfahren und mit üblichen technischen Geräten, wie Sprühtrockner oder Wirbelschichtgranulatoren erfolgen.

Mit Hilfe der vorliegenden Erfindung werden die 6 Hauptkavapyrone in mikrodisperser Form und/oder in Form einer festen Lösung gebunden. Bei der peroralen Applizierung der Kava-Extrakte werden dann die genannten 6 Hauptkavapyrone in optimaler Weise freigesetzt.

Die Fluidextrakte können auf einfache Weise dadurch hergestellt werden, daß das pulverisierte Rhizom von Piper methysticum Forst. (Rhizoma Kava-Kava) mit einem geeigneten Lösungsmittel extrahiert wird. Vorzugsweise wird das Lösungsmittel ausgewählt aus Aceton, Chloroform, Ethylacetat und C₁-C₄-Alkoholen, deren Gemischen untereinander und mit Wasser. Besonders bevorzugte Lösungsmittel sind Methanol, Ethanol, und Isopropanol sowie deren wasserfreie oder wasserhaltigen Gemische und insbesondere Ethanol/Wasser-Gemische. Die Extraktion kann in an sich bekannter Form einer Perkolation oder einer mehrstufigen Rühr- oder Wirbelextraktion bei Raumtemperatur oder bei erhöhter Temperatur durchgeführt werden. Je nach dem verwendeten Lösungsmittel weisen die so erhaltenen Rohextrakte einen Gehalt an Kavalactonen von etwa 40 bis 80 Gew.-% auf. Neben den genannten Lösungsmitteln ist in gleicher Weise auch die Extraktion mit überkritischen Kohlendioxid für die Herstellung der Rohextrakte einsetzbar.

Unter Zusatz des Trägers wird dann gegebenenfalls unter Erhöhung der Temperatur und/oder unter Vakuum das Lösungsmittel des Extraktes abgezogen und bis auf einen festen oder halbfesten Rückstand zur Trockne eingeengt.

In weiteren bevorzugten Ausführungsformen ist es erfindungsgemäß bevorzugt, die Kava-Fluidextrakte mit weiteren Hilfs- und/oder Zusatzstoffen vor dem vollständigen Einengen zur Trockne in Kontakt zu bringen. In gleicher Weise kann es, je nach Auswahl der weiteren Hilfs- und/oder Zusatzstoffe von Vorteil sein, die bereits zur Trockne eingeengten Kava-Fluidextrakte mit weiteren Hilfs- und/oder Zusatzstoffen in Kontakt zu bringen. Insbesondere feinteilige Hilfsstoffe, wie Kieselsäure werden vorzugsweise erst nach dem Einengen der Kava-Fluidextrakte mit diesen in Kontakt gebracht.

### Beispiele:

### Beispiel 1:

In 10 kg Kava-Fluidextrakt (Auszugsmittel Ethanol 96%, Gehalt Kavapyrone 2%) wurden 670 g Polyvinylpyrrolidon (Kollidon ® 25 (MW 25.000) gelöst. Nach dem Einengen unter Vakuum entstand ein fester Rückstand, der nach dem Zerkleinern mit Lactose und Magnesiumstearat zu Tabletten verpreßt wurde.

Die Zusammensetzung der Tablette mit 120 mg Kavapyronen war wie folgt:

| | |
|---|---|
| Rückstand | 648 mg (= 120 mg Kavapyrone), |
| Lactose | 50 mg und |
| Magnesiumstearat | 10 mg. |

### Beispiel 2:

240 g Kava-Spissumextrakt (50% Kavapyrone) wurden in 500 g Äthanol 96% unter Erwärmen gelöst und mit 400 g Polyvinylpyrrolidon (Kollidon^{R} MW 25.000) versetzt. Nach dem Auflösen des Polyvinylpyrrolidons wurde die alkoholische Phase in Vakuum eingeengt. Es entstand ein Rückstand, der anschließend mit Magnesiumstearat und Lactose zu Tabletten verpreßt wurde.

Die Zusammensetzung einer Tablette betrug 120 mg Kavapyrone entsprechend:

| | |
|---|---|
| Rückstand | 640 mg (=120 mg Kavapyrone), |
| Lactose | 50 mg und |
| Magnesiumstearat | 10 mg. |

### Beispiel 3:

Der gemäß Beispiel 1 erhaltene Rückstand wurde analog mit Cellulosepulver und Magnesiumstearat in Kapseln abgefüllt.

Die Zusammensetzung einer Kapsel mit 60 mg Kavapyrone ergab einen

| | |
|---|---|
| Rückstand | 324 mg (= 60 mg Kavapyrone), |
| Cellulosepulver | 50 mg und |
| Magnesiumstearat | 6 mg. |

Das Freisetzungsspektrum ist der Tabelle 1 zu entnehmen.

### Beispiel 4:

In 10 kg eines ethanolischen Kava-Fluidextrakts wurden (mit 2 Gew.-% Kavapyron) 0,6 kg Carboxymethylcellulose-Natrium suspendiert und anschließend das Lösungsmittel abgezogen. Es entstand ein Rückstand, der mit hochdispersem Siliciumdioxid (Aerosil® ) und mikrokristalliner Cellulose gemischt und anschließend nach Zusatz von Magnesiumstearat zu Tabletten verpreßt wurde.

Die Zusammensetzung einer halbfesten Tablette mit 120 mg Kavapyronen betrug

| | |
|---|---|
| Rückstand | 630 mg, |
| Hochdisperses Siliciumdioxid | 157 mg, |
| Mikrokristalline Cellulose | 154 mg und |
| Magnesiumstearat | 7 mg. |

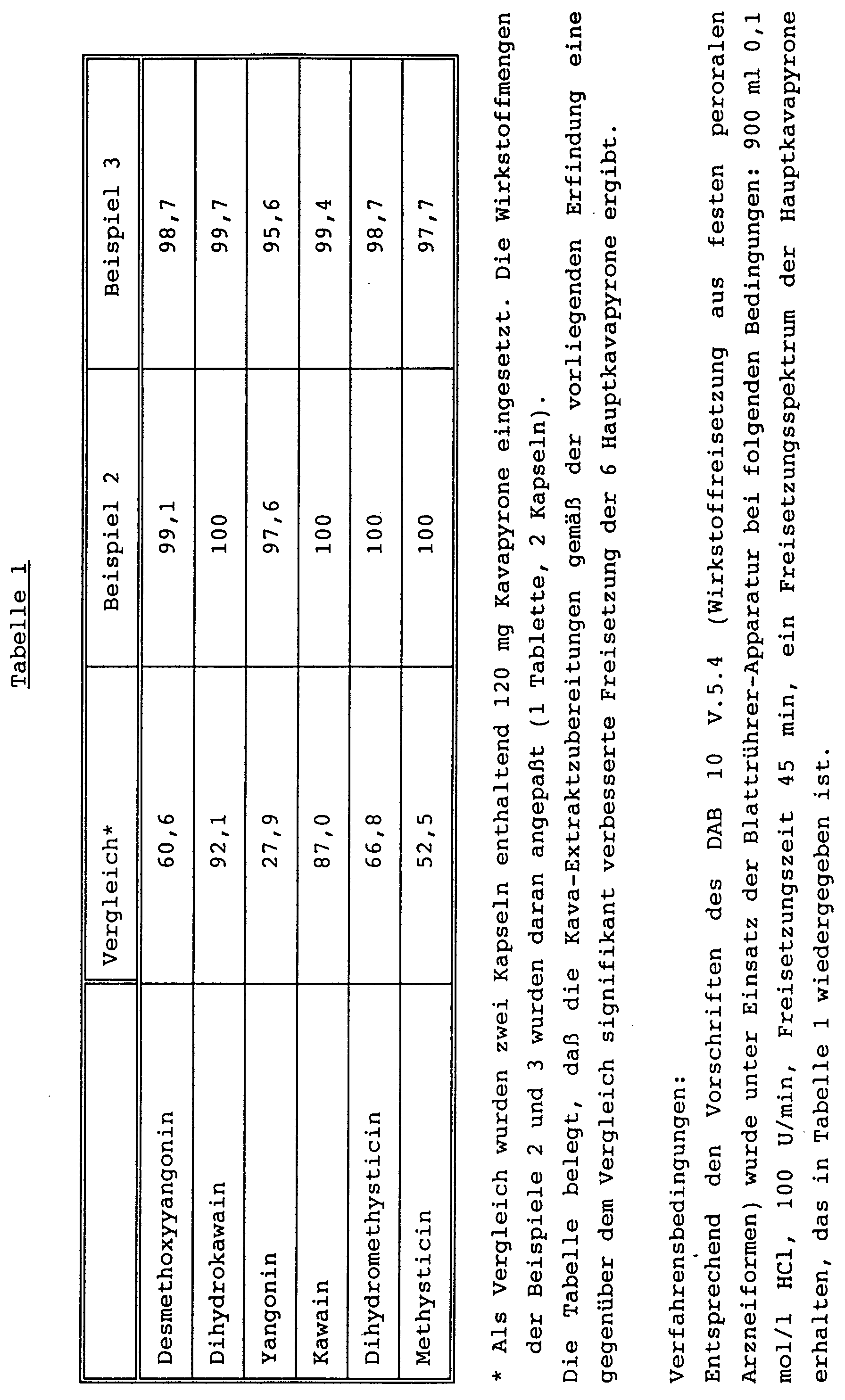

## Patentansprüche

1. Peroral applizierbare Kava-Extrakte, dadurch gekennzeichnet, daß sie Kavapyrone an bei Raumtemperatur feste Träger, ausgewählt aus Polyvinylpyrrolidon, Cellulosederivaten und/oder Stärkederivaten, in mikrodisperser Form und/oder in Form einer festen Lösung gebunden, gegebenenfalls neben weiteren Hilfs- und/oder Zusatzstoffen enthalten, **wobei das Gewichtsverhältnis von Kavapyronen zu Träger 1:1 bis 1:10 beträgt.**

2. Kava-Extrakte nach Anspruch 1, dadurch gekennzeichnet, daß die Cellulose- und/oder Stärkederivaten ausgewählt sind aus quervernetzten Cellulose- und/oder quervernetzten Stärkederivaten, insbesondere Alkalimetallsalzen von Carboxymethylstärke oder von Carboxymethylcellulose, insbesondere das Natriumsalz der Carboxymethylcellulose.

3. Kava-Extrakte nach **Anspruch 1 oder 2,** dadurch gekennzeichnet, daß **das Gewichtsverhältnis von Kavapyronen zu Träger 1:2 bis 1:6** beträgt.

4. Kava-Extrakte nach einem der Ansprüche 1 bis **3**, dadurch gekennzeichnet, daß die Hilfs- und/oder Zusatzstoffe ausgewählt sind aus Bindemitteln, Gleitmitteln, Füllstoffen, Emulgatoren, Netzmitteln und/oder Sprengmitteln.

5. Verfahren zur Herstellung von peroral applizierbaren Kava-Extrakten nach einem der Ansprüche 1 bis **4**, dadurch gekennzeichnet, daß man zu einem Kava-Fluidextrakt einen bei Raumtemperatur festen Träger, ausgewählt aus Polyvinylpyrrolidon, Cellulosederivaten und/oder Stärkederivaten zugibt und den Fluidextrakt bei gegebenenfalls erhöhter Temperatur und/oder unter Vakuum bis zur Trockne einengt.

6. Verfahren nach Anspruch **5**, dadurch gekennzeichnet, daß man einen Kava-Fluidextrakt hergestellt mit Hilfe eines Lösungsmittels ausgewählt aus Aceton, Chloroform, Ethylacetat und C₁-C₄-Alkoholen, insbesondere Methanol, Ethanol und Isopropanol sowie deren Gemische untereinander und mit Wasser einsetzt.

7. Verfahren nach Anspruch **5**, dadurch gekennzeichnet, daß den Fluidextrakt durch Sprühtrocknung oder in einem Wirbelschichtgranulator einengt.

8. Verfahren nach einem oder mehreren der Ansprüche **5 bis 7**, dadurch gekennzeichnet, daß man den Kava-Fluidextrakt mit weiteren Hilfs- und/oder Zusatzstoffen in Kontakt bringt.

9. Verfahren nach einem oder mehreren der Ansprüche **5 bis 7**, dadurch gekennzeichnet, daß man den eingeengten Kava-Fluidextrakt mit weiteren Hilfs- und/oder Zusatzstoffen in Kontakt bringt.

## Claims

1. Orally administrable kava extracts, characterized by containing kava pyrones bound, in a microdisperse form and/or in the form of a solid solution, to carriers which are solid at room temperature and selected from polyvinyl pyrrolidone, cellulose derivatives and/or starch derivatives, optionally in addition to other auxiliaries and/or additives, wherein the weight ratio of kava pyrones to carrier is from 1:1 to 1:10.

2. The kava extracts according to claim 1, characterized in that said cellulose and/or starch derivatives are selected from cross-linked cellulose and/or cross-linked starch derivatives, especially alkali metal salts of carboxymethylstarch or of carboxymethylcellulose, especially the sodium salt of carboxymethylcellulose.

3. The kava extracts according to claim 1 or 2, characterized in that the weight ratio of kava pyrones to carrier is from 1:2 to 1:6.

4. The kava extracts according to any of claims 1 to 3, characterized in that said auxiliaries and/or additives are selected from binders, lubricants, fillers, emulsifiers, wetting agents and/or disintegrants.

5. A method for the preparation of the orally administrable kava extracts according to any of claims 1 to 4, characterized in that a carrier which is solid at room temperature and selected from polyvinyl pyrrolidone, cellulose derivatives and/or starch derivatives is added to a kava fluid extract, and the fluid extract is concentrated to dryness, optionally at an elevated temperature and/or under vacuum.

6. The method according to claim 5, characterized in that a kava fluid extract prepared using a solvent selected from acetone, chloroform, ethyl acetate and C₁-C₄ alcohols, especially methanol, ethanol and isopropanol and their mixtures with one another and with water is employed.

7. The method according to claim 5, characterized in that the fluid extract is concentrated by spray-drying or in a fluidized-bed granulator.

8. The method according to one or more of claims 5 to 7, characterized in that the kava fluid extract is contacted with further auxiliaries and/or additives.

9. The method according to one or more of claims 5 to 7, characterized in that the concentrated kava fluid extract is contacted with further auxiliaries and/or additives.

## Revendications

1. Extraits de kava pouvant être administrés par voie orale, caractérisés en ce qu'ils contiennent des kavapyrones sur des supports solides à la température ambiante, choisis parmi la polyvinylpyrrolidone, les dérivés de la cellulose et/ou les dérivés de l'amidon, liées sous forme microdispersée et/ou sous forme d'une solution solide, éventuellement avec d'autres adjuvants et/ou additifs, le rapport en poids des kavapyrones au support étant de 1:1 à 1:10.

2. Extraits de kava selon la revendication 1, caractérisés en ce que les dérivés de la cellulose et/ou de l'amidon sont choisis parmi les dérivés de la cellulose à réticulation croisée et/ou les dérivés de l'amidon à réticulation croisée, en particulier parmi les sels de métaux alcalins du carboxyméthylamidon ou de la carboxyméthylcellulose, en particulier le sel de sodium de la carboxyméthylcellulose.

3. Extraits de kava selon la revendication 1 ou 2, caractérisés en ce que le rapport en poids des kavapyrones au support est de 1:2 à 1:6.

4. Extraits de kava selon l'une des revendications 1 à 3, caractérisés en ce que les adjuvants et/ou additifs sont choisis parmi les liants, les lubrifiants, les matières de charge, les émulsifiants, les mouillants et/ou les désintégrants.

5. Procédé de préparation d'extraits de kava pouvant être administrés par voie orale selon l'une des revendications 1 à 4, caractérisés en ce qu'on ajoute à un extrait fluide de kava un support solide à la température ambiante choisi parmi la polyvinylpyrrolidone, les dérivés de la cellulose et/ou les dérivés de l'amidon, et que l'on concentre l'extrait fluide jusqu'à siccité, à une température éventuellement élevée et/ou sous vide.

6. Procédé selon la revendication 5, caractérisé en ce qu'on utilise un extrait fluide de kava préparé à l'aide d'un solvant choisi parmi l'acétone, le chloroforme, l'acétate d'éthyle et les alcools en C₁ à C₄, en particulier le méthanol, l'éthanol et l'isopropanol, ainsi que leurs mélanges les uns avec les autres et avec l'eau.

7. Procédé selon la revendication 5, caractérisé en ce qu'on concentre l'extrait fluide par séchage par atomisation ou dans un granulateur à lit fluidisé.

8. Procédé selon une ou plusieurs des revendications 5 à 7, caractérisé en ce qu'on met en contact l'extrait fluide de kava avec d'autres adjuvants et/ou additifs.

9. Procédé selon une ou plusieurs des revendications 5 à 7, caractérisé en ce qu'on met en contact l'extrait fluide de kava concentré avec d'autres adjuvants et/ou additifs.
